(19) Europäisches Patentamt
European Patent Office
Office européen des brevets

(11) Numéro de publication: **0 115 714**
**A1**

(12) **DEMANDE DE BREVET EUROPEEN**

(21) Numéro de dépôt: **83402169.3**

(22) Date de dépôt: **09.11.83**

(51) Int. Cl.³: **C 07 D 239/46**, A 61 K 31/505

(30) Priorité: **28.01.83 FR 8301367**

(43) Date de publication de la demande: **15.08.84 Bulletin 84/33**

(84) Etats contractants désignés: **AT BE CH DE FR GB IT LI LU NL SE**

(71) Demandeur: **SANOFI, société anonyme, 40, Avenue George V, F-75008 Paris (FR)**

(72) Inventeur: **Carminati, Paolo, Via Pacini 24, Milan (IT)**
Inventeur: **Biziere, Kathleen, 6 Lotissement Rayons d'Oc, F-34170 Clapiers (FR)**
Inventeur: **Hallot, André, 83 rue du Juge, F-34980 Saint Gely du Fesc (FR)**

(74) Mandataire: **Combe, André et al, CABINET BEAU DE LOMENIE 55 rue d'Amsterdam, F-75008 Paris (FR)**

# (54) Composition pharmaceutique à action psychotrope dopanimergique.

(57) Composition pharmaceutique à action psychotrope dopaminergique renfermant, en tant que principe actif, une 2-pipérazinopyrimidine de formule

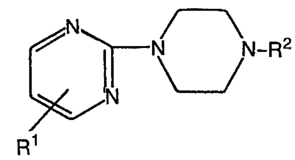

dans laquelle $R^1$ est de l'hydrogène ou un groupe hydroxyle et $R^2$ est de l'hydrogène ou un groupe alkyle inférieur; ou un de ses sels pharmaceutiquement acceptables en mélange avec un excipient pharmaceutique.

EP 0 115 714 A1

Composition pharmaceutique à action psychotrope dopaminergique.

---

La présente invention concerne une composition pharmaceutique à action psychotrope dopaminergique.

Plus particulièrement, l'invention se réfère à une composition pharmaceutique renfermant, en tant que principe actif, une 2-pipérazinopyrimidine de formule

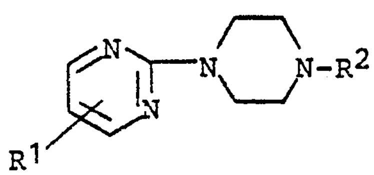

I

dans laquelle $R^1$ représente de l'hydrogène ou un groupe hydroxyle et $R^2$ représente de l'hydrogène ou un groupe alkyle contenant de 1 à 6 atomes de carbone; ou un de ses sels pharmaceutiquement acceptables.

Les composés de formule I dans laquelle $R^1$ est H ou OH et $R^2$ est H sont connus, en littérature, comme intermédiaires chimiques de synthèse.

La 2-(1-pipérazinyl)pyrimidine a été testée parmi toute une série de composés dont aucun n'a montré d'activité analgésique ou antifilariale (H.W. Stewart et al., J. Org. Chem. 1953, 18, 1478).

Son sel d'addition avec l'acide chlorhydrique est décrit dans le brevet indien 147 985 comme intermédiaire dans la synthèse de dérivés de la tétracycline.

On a maintenant trouvé que les composés de formule I ci-dessus ainsi que leur sels pharmaceutiquement acceptables

BAD ORIGINAL

possèdent une très bonne activité psychotrope avec un mécanisme d'action dopaminergique, notamment une activité antipsychotique, antidépressive et tranquillisante-sédative, qui leur permet d'être utilisés comme médicaments dans des compositions pharmaceutiques pour le traitement des affections psychiques, neurologiques et neuromusculaires des mammifères, y-compris l'être humain.

L'activité des composés de la présente invention a été évaluée dans les tests prédictifs des activités antipsychotique, antidépressive et tranquillisante-sédative et du mécanisme d'action dopaminergique.

L'activité antipsychotique d'un composé représentatif, le monomaléate de 2-(1-pipérazinyl)pyrimidine, a été déterminée dans le test de l'antagonisme de la toxicité de groupe due à l'amphétamine (J.H. Burn et al., Arch. Int. Pharmacodyn. Ther. 1958, 113, 290).

Le monomaléate de 2-(1-pipérazinyl)pyrimidine a été administré à des doses croissantes par voie intrapéritonéale à des lots de 10 souris Charles River CD1 pesant 20-22 g et groupées dans des cages de 22 cm de long, 12 cm de large et 12 cm de haute. Trente minutes après l'administration, on a injecté par voie intrapéritonéale de l'amphétamine à la dose de 30 mg/kg. La mortalité des animaux a été évaluée durant les 24 h qui ont suivi l'injection d'amphétamine et comparée, dans chaque groupe, à celle du groupe d'animaux témoins n'ayant reçu que le véhicule (sérum physiologique).

Le tableau I ci-dessous montre la dose efficace médiane (DE50) d'antagonisme de la mortalité due à l'amphétamine.

BAD ORIGINAL

TABLEAU I

| Composé | DE50 (limites de confiance) |
|---|---|
| 2-(1-pipérazinyl)pyrimidine monomaléate | 16,5 mg/kg (10,1 - 26,7) |

De ce tableau, il ressort que le composé de la présente invention possède une bonne activité dans le test de la toxicité de groupe due à l'amphétamine, prédictif d'une action antipsychotique.

L'activité antidépressive des composés de l'invention a été évaluée dans le test de l'antagonisme vis-à-vis de l'installation de la catalepsie provoquée par la prochlorpérazine (K. Bizière et al., Arzneimittel Forschung, 1982, 32 (II), 824).

Un composé représentatif de l'invention, à savoir le monomaléate de 2-(1-pipérazinyl)pyrimidine, a été administré par voie intrapéritonéale à des groupes de 10 rats mâles Wistar pesant 220-240 g; des animaux de contrôle ont été en même temps traités par du sérum physiologique. Une heure après, les animaux ont reçu, par voie sous-cutanée, de la prochlorpérazine à une dose de 10 mg/kg. Cinq heures après cette administration, le nombre d'animaux cataleptiques a été estimé par le test du bouchon. Ce test consiste à positionner les animaux de telle sorte qu'ils puissent maintenir pendant au moins 20 seconds leurs pattes en appui sur une colonne formée de trois bouchons de liège superposés

BAD ORIGINAL

(hauteur totale 11 cm). Les performances réalisées pour chaque groupe d'animaux ont été comparées à celles du groupe d'animaux témoins n'ayant reçu que le véhicule et la prochlorpérazine.

Le tableau II ci-dessous montre le pourcentage d'antagonisme de l'installation de la catalepsie provoquée par la prochlorpérazine.

TABLEAU II

| Composé | Dose | % d'antagonisme |
|---|---|---|
| 2-(1-pipérazinyl)pyrimidine | 0,1 mg/kg | 20 % |
| monomaléate | 1 mg/kg | 80 % |

De ce tableau, il ressort que le monomaléate de 2-(1-pipérazinyl)pyrimidine de la présente invention est actif dans le test de la catalepsie provoquée par la prochlorpérazine, prédictif d'une activité antidépressive.

L'activité tranquillisante-sédative des composés de l'invention a été déterminée dans le test de la motilité des souris selon la technique de J.R. Boissier et al.(Arch. Int. Pharmacodyn. 1965, 158, 212).

Un composé représentatif de l'invention, à savoir le monomaléate de 2-(1-pipérazinyl)pyrimidine, a été administré par voie intrapéritonéale à des lots de 12 souris par dose; en même temps, un lot d'animaux témoins n'a reçu que du sérum physiologique. Les animaux ont été placés individuellement 45 min après l'administration dans des cages actimétriques type Apelab (L = 26 cm, l = 21,5 cm, H = 10 cm)

BAD ORIGINAL

traversées par deux rayons lumineux qui impressionnent une cellule photo-électrique. Chaque traversée d'un faisceau lumineux a été comptabilisée par un compteur individuel. Les scores correspondant aux déplacements des animaux ont été enregistrés pendant 10 min.

Le tableau III ci-dessous montre la variation pour cent de la motilité spontanée des animaux traités aux différentes doses par rapport aux animaux témoins.

TABLEAU III

| Composé | Dose | Variation % |
|---|---|---|
| 2-(1-pipérazinyl)pyrimidine | 2,5 mg/kg | - 51 % ** |
| monomaléate | 1,25 mg/kg | - 39 % * |
| | 0,60 mg/kg | - 24 % ns |

** p < 0,01    * p < 0,05    Test "t" de Student

ns : non significatif

De ce tableau, il ressort que le monomaléate de 2-(1-pipérazinyl)pyrimidine de la présente invention manifeste une bonne activité sédative enregistrée par une baisse de la motilité spontanée de la souris.

Le mécanisme dopaminergique d'un ingrédient actif représentatif de l'invention, la 2-(1-pipérazinyl)pyrimidine chlorhydrate, a été étudié par l'analyse du comportement de rotation chez la souris après lésion unilatérale du striatum (P. Protais et al., J. Pharmacol. 1976, 7, 251).

BAD ORIGINAL

Des souris femelles Charles River CD1 pesant de 20 à 24 g ont préalablement fait l'objet d'une lésion unilatérale du striatum par injection stéréotaxique de 6-hydroxydopamine à raison de 8 mcg par animal. Une semaine après cette opération, le composé sous examen a été administré par voie orale à des groupes de 6 souris à une dose correspondant à 0,15 mg/kg de base libre. Le nombre de rotations à été évalué, pendant 2 minutes, 15 et 30 minutes après l'administration du produit. Les rotations ipsilatérales à la lésion ont été comptées positivement, celles contralatérales ont été comptées négativement. La somme algébrique des rotations pour chaque groupe d'animaux traités a été comparée à celle du groupe d'animaux témoins n'ayant reçu que le véhicule (sérum physiologique).

Le Tableau IV ci-dessous montre la variation pour cent, par rapport aux animaux témoins, des rotations.

TABLEAU IV

| Composé | Variation % après minutes: | |
|---|---|---|
| | 15 | 30 |
| 2-(1-pipérazinyl)-pyrimidine chlorhydrate | - 117 ** | - 58 ** |

** significatif $p < 0,01$ Test "t" de Student

De ce tableau il ressort que l'ingrédient actif des compositions pharmaceutiques de la présente invention réduit d'une façon significative la somme algébrique des rotations ipsilatérales et contralatérales à la lésion.

BAD ORIGINAL

L'activité psychotrope dopaminergique des composés de la présente invention et leur faible toxicité les rendent utiles comme médicaments.

Ainsi, la présente invention a pour objet des compositions pharmaceutiques renfermant les composés de formule I ci-dessus en tant qu'ingrédients actifs.

Dans les compositions pharmaceutiques de la présente invention pour l'administration orale, parentérale, sublinguale, transdermique ou rectale, les ingrédients actifs peuvent être administrés sous formes unitaires d'administration, en mélange avec des supports pharmaceutiques classiques, aux animaux et aux êtres humains pour le traitement des troubles de l'humeur ou du comportement, notamment dans le cas de psychose, dépression, ainsi que des états anxieux et des insomnies. Parmi les formes unitaires d'administration appropriées, il y a les formes par voie orale telles que les comprimés, les gélules, les poudres, les granules et les solutions ou suspensions orales et les suppositoires pour une administration rectale.

Afin d'obtenir l'effet psychotrope désiré, la dose de principe actif peut varier entre 0,1 et 100 mg par kg de poids du corps et par jour.

Chaque unité de dosage peut contenir de 1 à 300 mg d'ingrédient actif en combinaison avec un support pharmaceutique. Cette unité de dosage peut être administrée 1 à 4 fois par jour pour le traitement des troubles de l'humeur ou du comportement.

BAD ORIGINAL

Les composés de formule I qui ne sont pas connus peuvent être aisement préparés par réaction d'une pyrimidine substituée en position 2 de formule

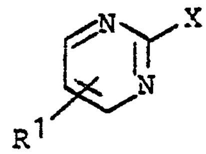

où X représente un atome de chlore ou un groupe méthylthio et $R^1$ est tel que défini ci-dessus, avec une pipérazine de formule

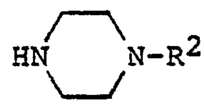

où $R^2$ est tel que défini ci-dessus, ou bien, lorsque $R^2$ est autre que l'hydrogène, par réaction d'une 2-pipérazino-pyrimidine de formule

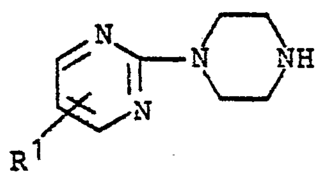

où $R^1$ est tel que défini ci-dessus, avec un halogénure d'alkyle selon des modes opératoires connus en soi.

Les exemples suivants illustrent l'invention sans toutefois la limiter.

EXEMPLE 1.

On chauffe au reflux pendant 18 heures un mélange de 3,2 g de 2-chloropyrimidine et de 12,1 g de pipérazine anhydre dans 100 ml d'éthanol absolu, puis on évapore à fond sous pression réduite et à chaud. On reprend le résidu avec 400 ml d'éther diéthylique, on lave la solution ainsi obtenue d'abord avec 10 ml d'une solution aqueuse d'hydroxyde de sodium 1:1, puis avec 20 ml d'eau. On sèche la solution

BAD ORIGINAL

organique sur du sulfate de sodium anhydre, on la filtre et on l'évapore à sec sous pression réduite. On dissout l'huile ainsi obtenue dans 30 ml d'isopropanol et on ajoute à la solution 2,7 g d'acide maléique dissous dans 30 ml d'isopropanol à 60°C environ. De la solution ainsi obtenue cristallise le produit qui est filtré et séché. On obtient 4 g de monomaléate de 2-(1-pipérazinyl)pyrimidine; F. 160-162°C. Par cristallisation ultérieure de l'éthanol à 95%, le point de fusion ne change pas.

EXEMPLE 2.

A une solution de 5,6 g de monomaléate de 2-(1-pipérazinyl)pyrimidine et de 10,6 g de carbonate de sodium dans 100 ml de diméthylsulfoxyde, on ajoute 2,1 ml de 2-bromopropane et on chauffe le mélange réactionnel à 100°C pendant 5 heures. On concentre le solvant sous pression réduite, on reprend le résidu dans de l'eau, on extrait trois fois à l'acétate d'éthyle, on sèche sur du sulfate de sodium anhydre, on filtre et on concentre sous vide. On chromatographie le résidu sur gel de silice, éluant acétate d'éthyle : méthanol 9:1 et on concentre les fractions contenant le seul produit désiré. On dissout le résidu dans de l'éther diéthylique et l'on traite la solution avec une solution de gaz chlorhydrique dans de l'éther diéthylique. Après évaporation, on recristallise le résidu de l'éthanol absolu. On obtient ainsi 2 g de chlorhydrate de 2-(4-isopropyl-1-pipérazinyl)pyrimidine; F. $>$ 200°C (avec sublimation).

EXEMPLE 3.

BAD ORIGINAL

On chauffe au reflux pendant 15 heures un mélange de 7 g de 4-hydroxy-2-méthylthiopyrimidine (Berichte, 1973, 106, 3058b) et 6,2 ml de 1-méthylpipérazine dans 125 ml de butanol, puis on laisse revenir le mélange réactionnel à la température ambiante, on filtre les cristaux et on les cristallise de l'éthanol absolu. On obtient ainsi 6,2 g de 4-hydroxy-2-(4-méthyl-1-pipérazinyl)pyrimidine; F. 180-182°C.

EXEMPLE 4.

En opérant comme décrit à l'Exemple 3, par réaction de la 4-hydroxy-2-méthylthiopyrimidine avec la pipérazine, on obtient la 4-hydroxy-2-(1-pipérazinyl)pyrimidine; F. > 260°C.

EXEMPLE 5.

On prépare des gélules ayant la composition suivante:

| | |
|---|---|
| 2-(1-pipérazinyl)pyrimidine | 15 mg |
| lactose | 120 mg |
| stéarate de magnésium | 5 mg |

en mélangeant intimement des charges des ingrédients ci-dessus et en versant le mélange dans des gélules de gélatine dure.

EXEMPLE 6.

On prépare des comprimés ayant la composition suivante:

| | |
|---|---|
| chlorhydrate de 2-(1-pipérazinyl)pyrimidine | 20 mg |
| lactose | 100 mg |
| cellulose microcristalline | 30 mg |
| amidon de mais séché | 40 mg |
| stéarate de magnésium | 5 mg |

BAD ORIGINAL

en broyant l'ingrédient actif jusqu'à une dimension particulaire de 0,4 mm, en le faisant passer à travers un tamis de 0,4 mm d'ouverture de maille, en mélangeant la matière broyée avec les autres constituants et en comprimant pour former les comprimés.

De la même façon, on prépare des comprimés contenant 40 mg d'ingrédient actif.

EXEMPLE 7.

En opérant comme décrit dans l'Exemple 6 ci-dessus, on prépare des comprimés ayant la composition suivante:

| | |
|---|---|
| monomaléate de 2-(1-pipérazinyl)pyrimidine | 50 mg |
| lactose | 95 mg |
| amidon de mais | 100 mg |
| talc | 4,5 mg |
| stéarate de magnésium | 0,5 mg |

EXEMPLE 8.

On prépare des suppositoires ayant la composition suivante:

| | | |
|---|---|---|
| 2-(1-pipérazinyl)pyrimidine | | 50 mg |
| lactose | | 250 mg |
| masse pour suppositoires | q.s.p. | 1,7 g |

On mélange la substance active avec le lactose et on met le mélange uniformement en suspension dans la masse pour suppositoires fondue. On verse la suspension dans des moules refroidis pour former des suppositoires d'un poids de 1,7 g.

BAD ORIGINAL

REVENDICATIONS

1. Composition pharmaceutique à action psychotrope dopaminergique renfermant, en tant qu'ingrédient actif, une 2-pipérazinopyrimidine de formule

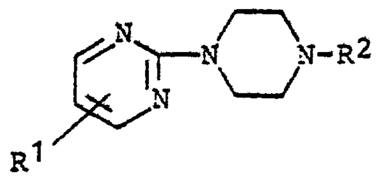

dans laquelle $R^1$ est de l'hydrogène ou un groupe hydroxyle et $R^2$ est de l'hydrogène ou un groupe alkyle contenant de 1 à 6 atomes de carbone; ou un de ses sels d'addition d'acides pharmaceutiquement acceptables.

2. Composition pharmaceutique selon la revendication 1, sous forme d'unité de dosage.

3. Composition pharmaceutique selon la revendication 2, caractérisée en ce qu'elle contient, pour chaque unité de dosage, de 1 à 300 mg d'ingrédient actif en mélange avec un excipient pharmaceutique.

BAD ORIGINAL

R E V E N D I C A T I O N (AT)

Procédé de préparations de dérivés de la Z-pipérazino-pyrimidine de formule

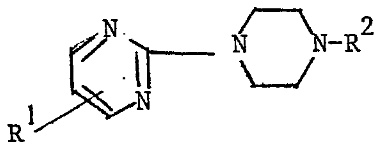

dans laquelle $R^1$ est de l'hydrogène ou un groupe hydroxyle et $R^2$ est de l'hydrogène ou un groupe alkyle contenant de 1 à 6 atomes de carbone et des sels d'addition d'acides pharmaceutiquement acceptables, lesdits dérivés étant utilisables comme produits à action psychotrope dopaminergique caractérisé en ce que on fait réagir une pyrimidrîne substituée en position L de formule

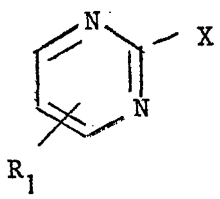

dans laquelle X représente un atome de chlore ou un groupe méthy-thio et $R_1$ est tel que défini ci-dessus, avec une pipérazine de formule

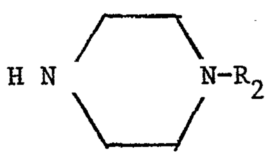

ou $R_2$ est tel que désigné ci-dessus.

Office européen des brevets

**RAPPORT DE RECHERCHE EUROPEENNE**

0115714

Numéro de la demande

EP 83 40 2169

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| X | JOURNAL OF CHROMATOGRAPHY, vol. 252, 1982, pages 310-314, Elsevier Scientific Publishing Company, Amsterdam, NL. S. CACCIA et al.: "Identification and quantitation of 1-(2-pyrimidinyl)piperazine, an active metabolite of the anxiolytic agent buspirone, in rat plasma and brain" * Pages 310-314 * --- | 1-3 | C 07 D 239/46<br>A 61 K 31/505 |
| X | US-A-2 562 036 (M. HULTQUIST) * Colonnes 1-6, exemple 7 * --- | 1,2 | |
| A | FR-M- 4 770 (SCIENCE UNION) * Pages 1-4 * ----- | 1,2 | |
| | | | DOMAINES TECHNIQUES RECHERCHES (Int. Cl. 3) |
| | | | C 07 D 239/00<br>A 61 K 31/505 |

Le présent rapport de recherche a été établi pour toutes les revendications

| Lieu de la recherche | Date d'achèvement de la recherche | Examinateur |
|---|---|---|
| LA HAYE | 05-05-1984 | FRANCOIS J.C.L. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82